# EUROPÄISCHE PATENTSCHRIFT

(11) **EP 1 088 212 B1**
(45) Veröffentlichungstag und Bekanntmachung des Hinweises auf die Patenterteilung: **21.11.2001**
(21) Anmeldenummer: 98928317.1
(22) Anmeldetag: 25.05.1998
(51) Int. Cl.: G01N 1/08, C12Q 1/68

(54) **VORRICHTUNG UND VERFAHREN ZUR GEWINNUNG UND ERST-AUFBEREITUNG VON GEWEBEPROBEN FÜR DIE MOLEKULARGENETISCHE DIAGNOSTIK**
DEVICE AND METHOD FOR OBTAINING AND INITIALLY PREPARING TISSUE SAMPLES FOR MOLECULAR GENETIC DIAGNOSIS
PROCEDE ET DISPOSITIF POUR PRELEVER ET SOUMETTRE A UN TRAITEMENT PREALABLE DES ECHANTILLONS TISSULAIRES A DES FINS DE DIAGNOSTIC RELEVANT DE LA GENETIQUE MOLECULAIRE

(43) Veröffentlichungstag der Anmeldung: 04.04.2001
(73) Patentinhaber: Agrobiogen GmbH, Larezhausen, 86567 Hilgertshausen (DE)
(72) Erfinder: BREM, Gottfried, D-86567 Hilgertshausen (DE)
(74) Vertreter: Becker Kurig Straus
(86) Internationale Anmeldenummer: EP9803075
(87) Internationale Veröffentlichungsnummer: WO9961882

(56) Entgegenhaltungen:
- EP-A- 0 734 768
- WO-A-96/13214
- GB-A- 2 137 340
- US-A- 4 230 001
- US-A- 5 126 276
- US-A- 5 396 898
- US-A- 5 741 177

## Beschreibung

Die vorliegende Erfindung betrifft eine Vorrichtung und ein Verfahren zur Gewinnung und Erst-Aufbereitung von Gewebe/Blut oder anderen Proben mit Kern- bzw. DNA-haltigen Zellen oder Zellbestandteilen für die molekulargenetische Untersuchung. Die Erfindung betrifft weiter die Verwendung der hier beschriebenen Vorrichtung zur Typisierung von Tierpopulationen.

Aus der EP-A-0734768 ist ein Verfahren zur Bindung eines biologischen Materials bekannt, wobei ein Probengewinnungsmittel in einen Probenaufnahmebehälter eingeführt und darin mittels Einrastrippen irreversibel fixiert wird.

Für diverse Forschungs- und Anwendungsprogramme ist die Gewinnung einer großen Anzahl von Gewebe- bzw. DNA-Proben notwendig. Dabei müssen unter bestimmten Umständen Informationen über ganzen (Nutztier)-Populationen oder regionale bzw. speziell charakterisierte Tierbestände (z.B. biologisch dynamische Tierproduktion) gewonnen werden.

So wurde beispielsweise ausgelöst durch den BSE-Skandal und die damit verbundenen Probleme die Herkunft von Fleisch und Produkten aus unbelasteten Betrieben zuzusichern eine EU-weite Kennzeichnungspflicht von Nutztieren eingeführt, wobei die Tiere bei oder kurz nach ihrer Geburt zur deren Kennzeichnung mit Ohrmarken versehen werden. Diese sind mit einer individuellen Nummer versehen, die das Nutztier kennzeichnen. So läßt sich durch spätere Überprüfung der Nummer beispielsweise im Schlachthof feststellen, aus welchen Betrieb das Tier stammt.

Diese Ohrmarken sind jedoch nicht fälschungssicher und können durch Manipulation ausgetauscht werden, so daß das eingeführte System der Kennzeichnung umgangen werden kann. Vom Verbraucher oder Zwischenhändler kann somit nicht sichergestellt werden, ob das Tier wirklich aus dem angegebenen Betrieb stammt.

Es wäre daher wünschenswert über ein einfaches analytisches Verfahren zu verfügen, das eine unabhängige Überprüfung der von Produzenten, Verarbeitern und Vermarktern gemachten Angaben ermöglicht.

Jedes Subjekt kann bekanntermaßen durch Untersuchung bestimmter DNA-Varianten als Individuum identifiziert werden ("genetischer Fingerabdruck"). In der Forensik, beispielsweise der Bestimmung eines Straftäters, und bei der Abstammungssicherung von Zuchttieren werden diese innovativen molekulargenetischen Techniken bereits analytisch genutzt, wobei normalerweise eine Probe des Subjekts über Blutentnahme durch den Arzt gewonnen und der Analyse zugeführt wird. Dies ist jedoch für größere Populationen, insbesondere bei großen Tierbeständen zu aufwendig und hinsichtlich der damit einhergehenden Kosten vom ökonomischen Standpunkt nicht durchführbar.

Mittels moderner Nachweisverfahren (PCR, Sequenzierung, usw) läßt sich gegenwärtig bereits an kleinsten Gewebeproben nachweisen, ob diese von einem bestimmten Individuum stammen oder nicht. Der Nachweis kann relativ einfach mit einer Zuverlässigkeit von über 99,9% geführt werden.

Der personelle und finanzielle Aufwand für eine gezielt und separat durchgeführte Gewinnung, Asservierung, Katalogisierung, Archivierung und Analyse solcher Probenmengen ist jedoch zur Zeit enorm.

Eine Aufgabe der vorliegenden Erfindung besteht daher darin eine Vorrichtung bereitzustellen, mit der eine Gewinnung von DNA-haltigen Proben aus einem Tier einfach und kostengünstig durchgeführt werden kann.

Eine weitere Aufgabe der Erfindung besteht darin ein Verfahren zu liefern, mit dem Gewebe- und/oder Blutproben aus Tieren einfach gewonnen und einem analytischen Verfahren zugeführt werden können.

Diese Aufgabe wird gelöst durch eine Vorrichtung zur Gewinnung und Erst-Aufbereitung von DNA-haltige Zellen enthaltenden Proben, gemäß Anspruch 1.

Die vorstehende Aufgabe wird weiter durch ein Verfahren gemäß Anspruch 15 gelöst.

Die Erfindung wird nun anhand der beiliegenden Zeichnungen erläutert, in denen:

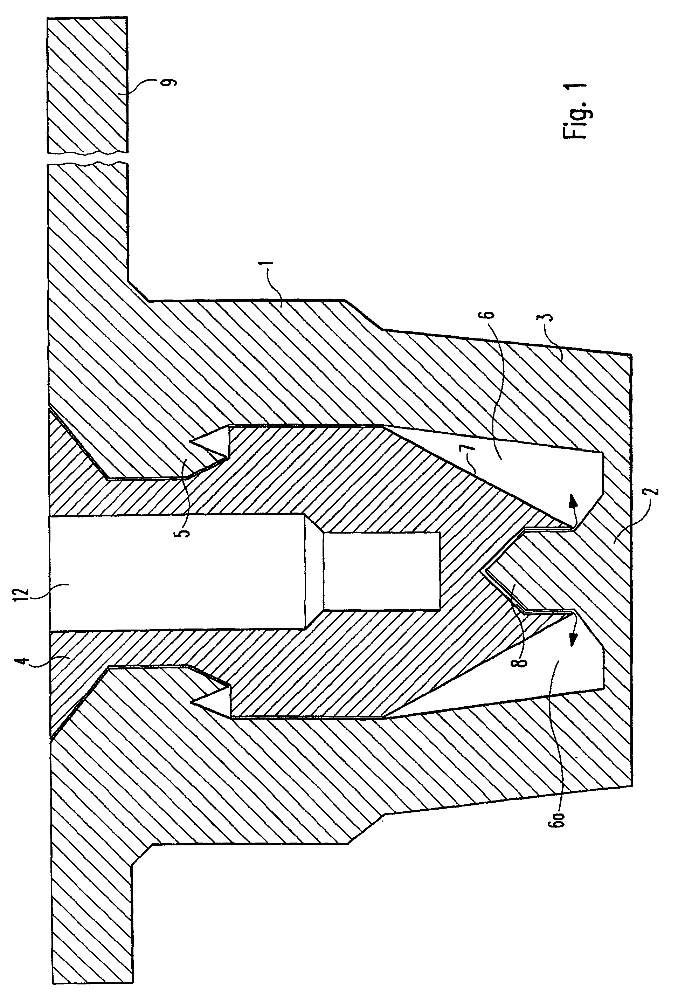

Fig. 1 einen Querschnitt einer Ausführungsform des Probenaufnahmebehälters 1 zeigt, in dem an dessen Boden 2 ein Vorsprung 8 ausgebildet ist. Das Probengewinnungsmittel 4 befindet sich in dem Probenaufnahmebehälter 1 und verschließt diesen dichtend. An der dem Probenraum 6,6a abgewandten Seite des Probengewinnungsmittels 4 ist eine Vertiefung 12 zur Aufnahme eines Stiftes ausgebildet.

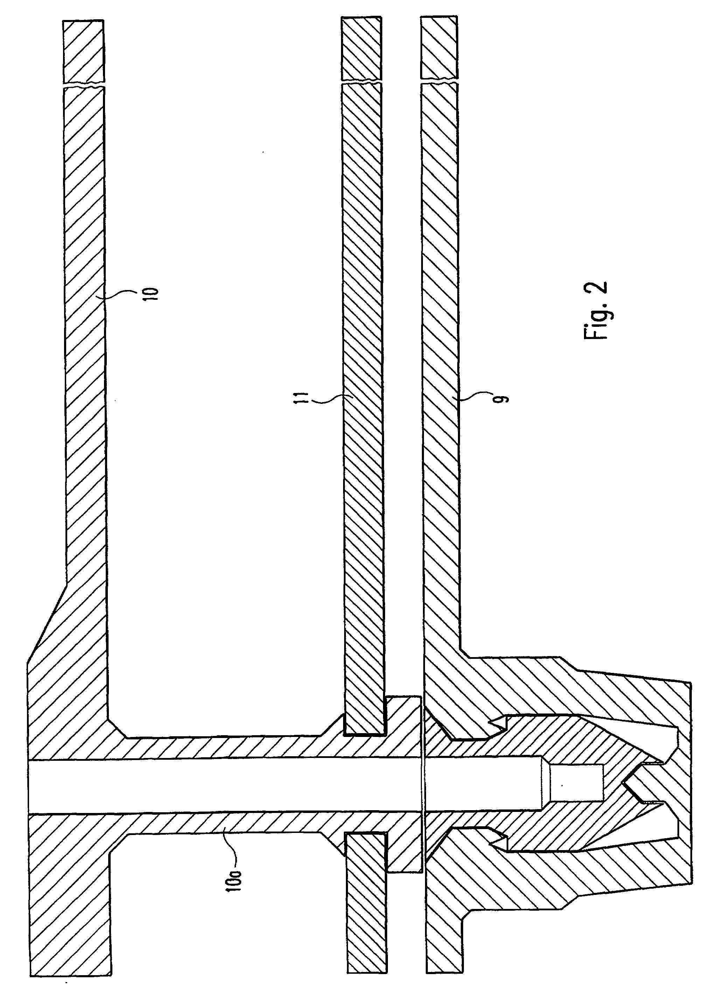

Fig. 2 einen Querschnitt einer Anordnung Dornplatte 10 mit Dorn 10a, Lochplatte 11 mit einem mit einer Lasche 9 ausgebildeten Probenaufnabmebehälter 1 zeigt, der mit dem Probengewinnungsmittel 4 verschlossen ist, wie sie sich nach Anbringen einer Ohrmarke 10,11 und gleichzeitiger Gewinnung einer Probe darstellt.

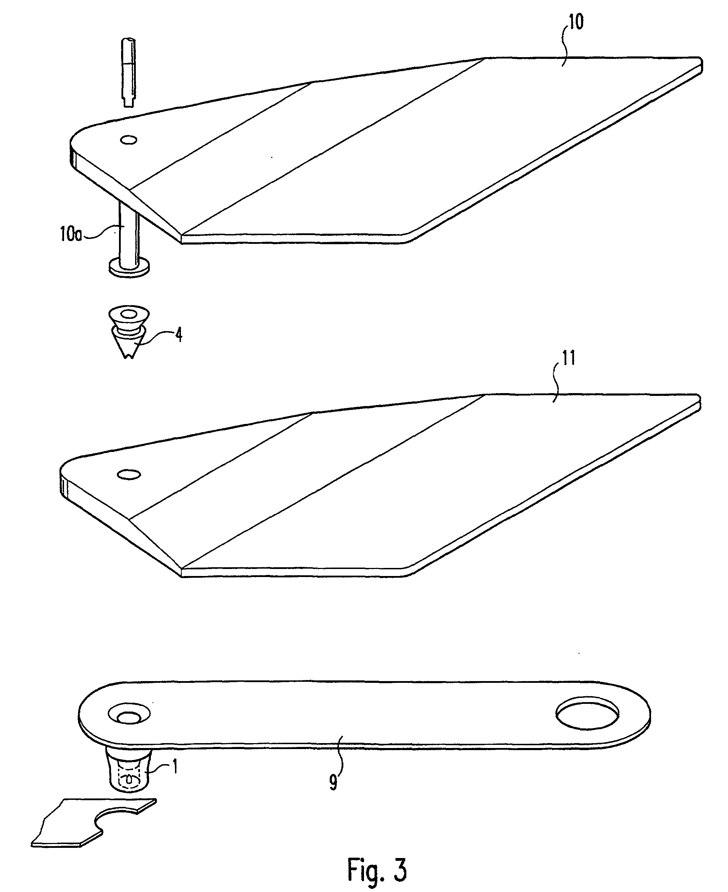

Fig. 3 eine Seitenansicht einer aus einer Dornplatte 10 und einer Lochplatte 11 bestehenden Ohrmarke 10,11 und eines mit einer Lasche 9 versehenen Probenaufnahmebehälters 1 vor Zusammemührung durch eine geeignete Vorrichtung darstellt.
Fig. 4 schematisch eine DNA-Individual-Typisierung beim Rind zeigt.

Die Erfindung wird nun anhand bevorzugter Ausführungsformen unter Bezugnahme auf die Zeichnungen ausführlich erläutert.

Die erfindungsgemäße Vorrichtung enthält einen Probenaufnahmebehälter 1 und ein Probengewinnungsmittel 4.

Der Probenaufnahmebehälter 1 weist einen Boden 2 und Seitenwände 3 auf und ist mit einem leicht durchdringbaren Deckel, wie einer Folie oder einer Membran verschlossen.

Der Probenaufnahmebehälter 1 kann selbst durch weitere Membranen unterteilt werden, so daß zwei oder mehr Komponenten im Probenaufnahmebehälter 1 bis zur Anwendung voneinander getrennt vorliegen und erst durch das Einbringen des Probengewinnungsmittels 4 in den Behälter 1 miteinander und mit der Probe in Kontakt kommen. Der Probenaufnahmebehälter 1 kann ferner durch eine Trennwand, wie einen sich über den gesamten Durchmesser des Bodens 2 erstreckenden Vorsprungs 8 in mindestens zwei Bereiche unterteilt sein, so daß bei einer Probengewinnung mindestens zwei voneinander getrennte Proben gewonnen werden können.

In einer bevorzugten Ausführungsform nimmt der Probenaufnahmebehälter 1 mindestens das vordere Ende des Probengewinnungsmittels 4 auf. Er kann im Bodenbereich 2 so ausgebildet sein, daß ein gegebenenfalls eine Kegelform aufweisender Vorsprung 8 vorhanden ist, wobei das Probengewinnungsmittel 4 zur Aufnahme des Vorsprungs 8 entsprechend angepaßt ist. Dies führt dazu, daß beim Einführen des Probengewinnungsmittel 4 in den Probenaufnahmebehälters 1 die Probe durch das Zusammenpressen der beiden Teile sehr stark zerquetscht und zerkleinert und in den durch den Boden 2 und die Seitenwände 3 des Probenaufnahmebehälters 1 sowie dem vorderen Bereich 7 des Probengewinnungsmittels 4 begrenzten Probenraum 6, 6a gedrückt wird.

Der Probenaufnahmebehälter 1 kann weiter eine Haltevorrichtung 9, wie ein flach angesetztes Teil, beispielsweise eine Lasche mit einem Loch, aufweisen, um diesen entprechend zu befestigen. So kann beispielsweise bei Verwendung einer Zange zum Anbringen der Ohrmarke 10,11 die Lasche an dieser befestigt werden. Die Lasche eignet sich weiter zur Aufnahme der Identifikationsnummer, die mit der der Ohrmarke übereinstimmt. Die Beschriftung kann von Hand erfolgen, in dafür vorgesehene vorgeformte Rillen eingetragen werden oder durch Vorbeschriftung, z.B. Einprägung, oder mit einem Drucker gleichzeitig und identisch mit dem Anbringen der Identifikationsnummer auf einem Plattenteil der Ohrmarke 10,11 erfolgen.

Der Probenaufnahmebehälter 1 kann gegebenenfalls (z.B. für eine Weiterbearbeitung der Proben von Hand) auch so ausgebildet sein, daß der Bereich des Probenaufnahmebehälters 1, der nach Verschließen mit dem Probengewinnungsmittel 4 den Probenraum 6,6a definiert, mit dem Korpus des Probenaufnahmebehälters 1 durch ein Gewinde verbunden ist und daher leicht abgeschraubt werden kann, so daß ein leichter Zugang zu der Probe ermöglicht wird.

In einer bevorzugten Ausführungsform wird der Probenaufnahmebehälter 1 mit dem Probengewinnungsmittel 4 derart verschlossen, daß ein Öffnen des gebildeten Probenraums 6,6a ohne Zerstörung der Vorrichtung nicht möglich ist. Dadurch kann sichergestellt werden, daß eine Manipulation der einmal gewonnenen Probe, ohne daß es bemerkt wird, nicht erfolgen kann.

In dem Probenaufnahmebehälter 1 befindet sich Material zur Inaktivierung des Proteinanteils der Gewebeprobe und zur Stabilisierung der DNA.

Das Material zur Inaktivierung von Proteinen (Enzyme, DNAsen usw.) der Gewebeprobe und zur Stabilisierung der DNA kann beispielsweise ausgewählt werden aus der Gruppe bestehend aus:
- Proteinase K (z.B. zur Stabilisierung während der Lagerdauer lyophilisert) und (getrennt eingefülltem) Puffer zum Verdau von Proteinen;
- starke Lauge;
- Molekularsieb (z.B. E. Merck 0,2 nm Nr. 1.05704.0250, K 230045904 624, Wasseraufnahmevermögen > 20%), das extrem hygroskopisch ist und bei Kontakt mit der Gewebeprobe diese austrocknet (und erwärmt) und damit inaktiviert. Zum Schutz des Molekularsiebes vor unerwünschter Aufnahme von Feuchtigkeit aus der Luft kann es z.B. mit dem Edelgas Argon überschichtet und mit einer Folie abgedeckt werden;
- anderen Bestandteilen, die die Inaktivierung des Proteinanteils und die Stabilisierung der DNA unterstützen.

Das Material wird so formuliert, daß es für einen langen Zeitraum, beispielsweise bis zu 1 Jahr oder länger aktiv bleibt und nach dem Einbringen der Probe zumindest für mehrere Monate bis zu einem Jahr eine hinreichende Integrität der DNA für eine analytische Untersuchung gewährt.

Das Probengewinnungsmittel 4 kann jede Form annehmen, mit der die Probe gewonnen und in den Probenaufnahmebehälter 1 eingeführt werden kann, wobei das Probengewinnungsmittel 4 den Probenaufnahmebehälter 1 nach Einführen dichtend verschließt. Dies schließt die Form von Zylindern, Kegeln, usw. ein.

Das Probengewinnungsmittel 4 kann massiv sein oder an dessen hinteren Ende eine Vorrichtung zur Aufnahme eines Stiftes, beispielsweise einen zentrischen Hohlraum 12, aufweisen, in den beim Benutzen ein stahlstift einer Zange zur Stabilisierung eingefuührt wird.

Mit der erfindungsgemäßen Vorrichtung wird das Ohr eines Tiers durchstochen, wobei das vordere Ende des Probengewinnungsmittel 4 scharfkantig ausgebildet sein kann, so daß beim Durchdringen des Ohres eine kleine Gewebeprobe ausgestanzt/gequetscht wird.

Das Probengewinnungsmittel wird zur Gewinnung der Probe mit einer geeigneten Vorrichtung, wie einer Ohrzange, gleichzeitig mit einer Ohrmarke durch das Ohr eines Subjekts gedrückt, so daß das Anbringen der Ohrmarke und die Gewinnung der Probe in einem Arbeitsgang vonstatten geht.

Die für die Markierung verwendete Ohrmarke besteht in der Regel aus zwei Teilen, einer den Dorn tragenden Platte 10 (Dornplatte) und einer Lochplatte 11, die etwa die gleiche Größe aufweist wie die Dornplatte 10 oder auch aus einer kleineren Scheibe bestehen kann, die nur groß genug sein muß um ein Herausrutschen des Dorns 10a aus dem Ohr zu verhindern. Beide Teile werden aus lebensmitteltauglichen und gewebeverträglichem Kunststoff (z.B. Polyurethan - Desmopan 795 U von Bayer) oder zum Teil aus Metall (z.B. Edelstahl,. Messing, Bronze o.ä.) gefertigt.

Zum Einziehen der Ohrmarken 10,11 kann eine handelsübliche Ohrmarkenzange verwendet werden. Die meisten für diesen Zweck verfügbaren Zangen können gegebenenfalls durch ein kleines Zusatzteil sehr einfach so umgerüstet werden, so daß der Probenaufnahmebehälter 1 nach dem Einziehen der Ohrmarke 10,11 an der Zange hängen bleibt und dadurch leicht aufgenommen werden kann. Das Zusatzteil ist beispielsweise eine kleine Noppe, über die die Lochöffnung der Lasche 9 mit dem Probenaufnahmebehälter 1 gezogen wird. Nach dem Einziehen der Ohrmarke 10,11 ins Ohr und dem gegebenenfalls ruckartigen Herausziehen der Ohrmarke 10,11 aus den Halterungen der Zange hält die Noppe die Lasche 9 mit dem Probenaufnahmebehälter 1 an der Zange fest. Anschließend kann die Lasche sehr leicht über die Noppe gezogen und damit der Probenaufnahmebehälter 1 eingesammelt werden.

Das Probengewinnungsmittel 4 verschließt den Probenaufnahmebehälter 1 durch Einführen dichtend und wird durch Befestigungsmittel 5 ortsstabil fixiert, so daß ein Austreten von Probenmaterial und ein Eindringen von Fremdmaterial verhindert wird.

Der Probenaufnahmebehälter 1 sowie das Probengewinnungsmittel 4 können aus jedem geeigneten Material gefertigt sein, wie beispielsweise aus Metall oder aus ggf. glasfaserversärktem Kunststoff. Ist das Probengewinnungsmittel 4 aus Kunststoff gefertigt, so kann kann es beim Einziehen ins Ohr durch einen an der verwendeten Zange befindlichen innenliegenden Metallstift verstärkt werden.

Mit der erfindungsgemäßen Vorrichtung wird ermöglicht, die Probenentnahme bei Tieren gleichzeitig mit der üblicherweise ohnehin durchgeführten Identifikationsmarkierung mit Ohrmarken 10,11 ohne wesentlichen zusätzlichen Aufwand durchzuführen. Die daraus resultierenden Einsparungen sind sehr hoch.

Ein weiterer Vorteil der erfindungsgemäßen Vorrichtung gegenüber konventionellen Probenentnahme-Behältern ist die an den Probenaufnahmebehälter anschließende Haltevorrichtung 9, die die Form einer flachen Lasche annehmen, und die zur Beschriftung/Kennzeichnung der Probe verwendet werden kann. Üblicherweise muß die Beschriftung auf der meist runden Oberflächen von meist zylindrischen Gefäßen (z.B. Eppendorf Tubes) mehr oder weniger mühsam und eventuell schwer leserlich von Hand angebracht werden. Ein Einlesen dieser Nummern oder Daten und eine automatisierte Entnahme ist dabei schwer zu realisieren.

Das erfindungsgemäße System ist insbesondere dann von Vorteil, wenn Proben aus Material gewonnen werden müssen, das sich noch in einem Verbund befindet, so daß eine Probe normalerweise mit einem Gerät (Messer, scharfer Löffel, Skalpell etc.) entnommen werden muß. Bei Verwendung von wiederverwendbaren Geräten ist die Kontaminationsgefahr sehr groß, da bei der sehr sensitiven analytischen Maßnahmen, wie PCR (Polymerase-Ketten-Reaktion) schon einzelne Zellen zu Kontaminationen und damit zu falsch positiven Ergebnissen führen können. Bei Verwendung der erfindungsgemäßen Vorrichtung kommen ausschießlich solche Teile mit dem Probenmaterial in Berührung, die nur einmal verwendet werden.

Ein wesentlicher Vorteil der Erfindung besteht darin, daß die Probenaufnahmebehälter 1 vor dem Verteilen an die Nutzer bzw. Tierbesitzer gleichzeitig mit den Ohrmarken (Dornplatte 10, Lochplatte 11) beschriftet werden können und dadurch unverwechselbar die selbe Nummerierung tragen. Die Beschriftung kann z.B. mit einem Drucker erfolgen, mit dem im Falle einer Kunststoffohrmarke ein (schwarzer) Mastermix aufgetragen wird, der sich so stabil mit dem Kunststoff verbindet, daß er bei üblichem Gebrauch nicht verwischt oder entfernt werden kann. Weiter können die entsprechenden Teile eine individuelle Zahl-Prägung aufweisen.

Die mit der erfindungsgemäßen Vorrichtung gewonnenen Proben werden dann an eine zentrale Sammestelle gebracht und dort analysiert. Das Sammeln der Probenaufnahmebehälter 1 kann ohne besondere Vorgaben hinsichtlich Temperatur und Dauer des Transportes erfolgen. Die Probenaufnahmebehälter 1 können problem- und gefahrlos per Post, Kurier oder Sammelaktion ins Labor oder Archiv transportiert werden.

Die Aufarbeitung der Proben im Labor - Entnahme eines Aliquots, Analyse-Reaktion und Auswertung - kann vollautomatisch mit Roboter-gesteuerten Systemen erfolgen. Die Identifikationsnummer der Proben wird dabei über ein Lesegerät erfaßt und weiterverarbeitet. Durch die Vermeidung einer Dateneingabe per Hand kann die Fehlerquote vernachläßigbar gering gehalten werden.

Zur Bearbeitung bzw. Analyse der DNA aus den mit den erfindungsgemäßen Vorrichtungen gesammelten Proben werden die Probenaufnahmebehälter 1 so in ein Förderband eingelegt, daß ein Lesegerät die Identität der Probe aufnehmen kann. Anschließend wird eine Kanüle durch den Boden des Probenraums 6,6a eingestochen, Flüssigkeit zur Aufnahme der Probe injiziert und anschließend ein Aliqout entnommen. Die Einstichstelle kann anschließend wieder versiegelt werden, so daß der Probenaufnahmebehälter archiviert werden kann. Wurde der Probenraum 6,6a bereits vorab in geeigneter Weise unterteilt so befinden sich in der erfindungsgemäßen Vorrichtung noch weitere, zur Analyse einsetzbare Proben.

Die DNA-Isolierung kann durch die Verwendung eines Pipettier-Roboters (beispielsweise Biomec 2000, Beckman) und der DNA-Reinigung mittels Silikon-Partikeln (z.B. InstaGene® Matrix, BIO-RAD) automatisiert werden. Die Analyse-Reaktionen mit diesen Proben können ebenfalls automatisiert erstellt werden.

Mit dem hier beschriebenen System der einfachen Gewinnung von Proben und automatisierter Analyse lassen sich folgende Vorteile erzielen:
- Fälschungssicherer Herkunfts- und Identitätsnachweis für alle Rinder und Fleisch von diesen Rindern in allen Stufen der Vermarktung und Verarbeitung bis hin zum Verzehr und damit der Nachweis der Herkunft aus BSE-freien Herkünften
- Ermöglichung der Kontrolle und damit Stabilisierung gezielter Vermarktungsprogramme wie z.B. von Tieren aus der Bioproduktion aus der Haltung in Nationalparks oder mit speziellen Gütezeichen
- Überprüfung und Überwachung der Transportwege, -zeiten und -entfernungen für alle Rindertransporte
- Effiziente Grenzkontrollen und Nachverfolgung inländischer Rinder im EU-Raum und bei internationalen Exporten
- Unverwechselbares Kennzeichen aller verkauften Zuchtrinder mit jederzeit nachweisbarer Identität z.B. bei Auktionen, Ausstellungen etc.
- Lückenloser Abstammungsnachweis für alle geborenen Rinder und Aufdeckung von Fehl- oder Falschbelegungen
- Unzweifelhafte forensische Nachweise (auch noch bei verzehrtem Rindfleisch aus Mageninhalt in der Gerichtsmedizin)
- Unzweifelhafte Überwachung von Keulungsmaßnahmen und bei Seuchensanierungen
- Feststellung der Beimengungen von Rindfleisch in allen zur Untersuchung vorgelegten (verarbeiteten) Lebensmitteln, einschließlich Heimtierfutter
- Aufdeckung der Identität von Produzenten von Schlachtkörpern, in denen im Rahmen von Stichprobenuntersuchungen Behandlungen mit unerlaubten Hormonen oder Wachstumsförderern vorgenommen worden sind
- Herkunftsnachweis bei anderen Produkten aus der Rinderhaltung wie Fellen, Knochen, Hörnern, Klauen, Organpräparationen, Blut etc.
- Nachweis und Kontrolle der Tier- und Bestandsherkunft bei direkt vermarkteter Milch und Milchprodukten
- Exakte Erfassung aller Rinderbestände und Vermarktungswege
- Proben für detaillierteste Analysen von DNA-Polymorphismen, genetische Distanzmessungen und genetische Screening-Programme würden zur Verfügung stehen
- Optimierung von Zuchtprogrammen
- Genetische Distanzmessungen
- Untersuchung der genealogischen und zuchthistorischen Entwicklung von Tierrassen
- Analysen im Rahmen Marker-gestützter-Selektionsprogramme (MAS)
- exakte Aussagen über die Präsenz und Häufigkeit bestimmter Erbfehlerallele

Aus forensischer Sicht hat die hier beschriebene Vorrichtung und das hier beschriebene Verfahren weiter den Vorteil, daß eine einmal eingepackte Probe nicht mehr verändert (ausgetauscht, verfälscht) werden kann, ohne daß die dazu notwendige Manipulation des Probenaufnahmebehälters erkennbar sein würde.

Durch Sammeln und Katalogisieren der gewonnenen Proben wird weiter eine Individualtypisierung, ein Herkunftsnachweis, Erbfehlers-Sreening, Populationsanalysen, Mutationsdetektion, sowie Lebensmittel-Screening, Diagnostik von Krankheitserregern, Transgenitätsdiagnostik, Überwachung der Hygienesituation in Betrieben der Lebensmittelver- und bearbeitung usw. ohne großen Aufwand ermöglicht.

Mit dem hier beschriebenen System wird ein Nachweis über die genetische Information, die nicht nur alle Tiere sondern auch die aus Tieren erstellten Produkte bis zum Verzehr enthalten, möglich. Mit der hier beschriebenen Vorrichtung können die normalerweise damit einhergehenden Kosten um ein Vielfaches gesenkt werden.

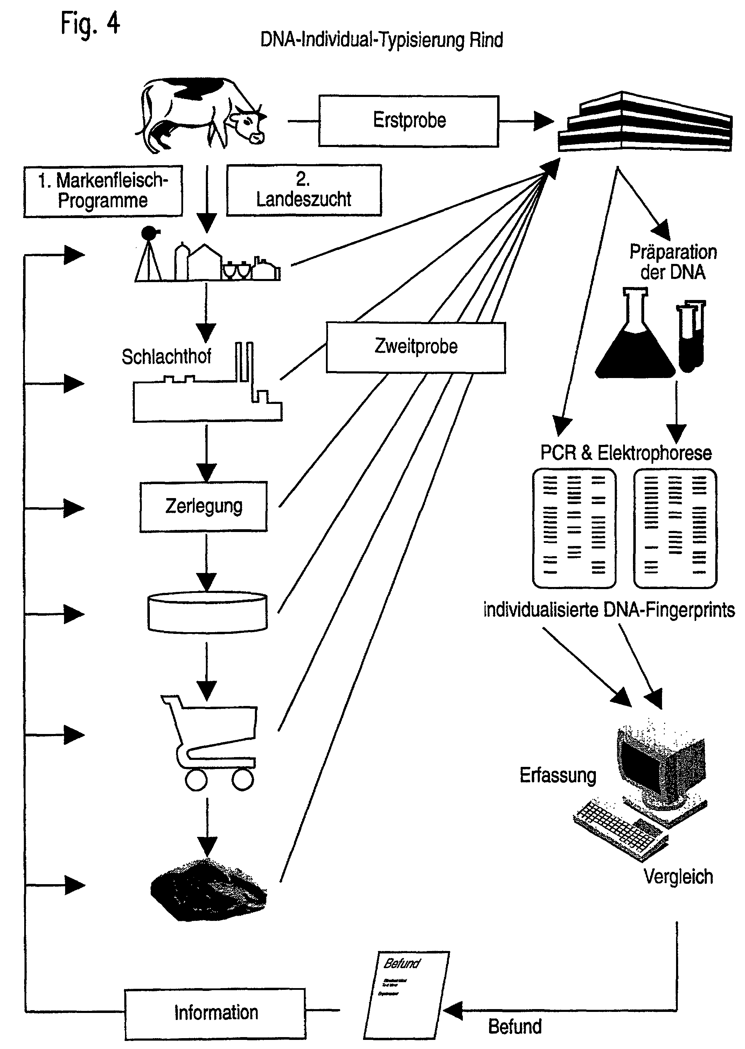

So kann sogar in intensiv bearbeiteten Lebensmitteln tierischen Ursprungs (Brühwürste, Bratenfleisch, Leberkäs, Schnitzel etc.) noch spezies- und individualspezifische DNA nachgewiesen werden. Dadurch wird ermöglicht, Kontaminationen von Lebensmitteln mit fremden Fleischanteilen nachzuweisen. Natürlich ist es mit Hilfe dieser Methoden auch möglich, zweifelsfrei zu beweisen, daß bestimmte Lebensmittel - wie angegeben - von einem bestimmten Tier stammen, wenn von dem lebenden Tier im Herkunftsbetrieb eine geeignete (Erst)Probe gezogen worden ist (Fig.4).

Dazu muß von jedem geborenen Nutztier eine Ohrgewebeprobe für die DNA-Typisierung gewonnen und an das Typisierungs-Zentrum übermittelt werden. Dort werden mittels (automatisierter) PCR DNA-Fingerprints mit Mikrosatelliten-Primern erstellt und so ein unverwechselbares Muster für jedes einzelne Tier erfaßt.

Diese Daten werden in einer Zentraldatei gespeichert und sind erschließbar über:
- den bei einer Kontrolluntersuchung aufgetretenen Fingerprint, d.h. bei Einsendung einer "Zweitprobe" kann nach der DNA-Typisierung durch Vergleich mit den Fingerprints aus der "Erstuntersuchung" das dazugehörige Tier herausgefunden werden. Dieses Ergebnis kann normalerweise innerhalb eines Tages bereitgestellt werden. In Extremfällen z.B. bei Tiertransporten oder Grenzkontrollen kann innerhalb von drei Stunden eine Express-Auskunft erteilt werden.
- die Tiemummer, d.h. von jedem erfaßten Tier sind der DNA-Fingerprint und alle anderen Daten abrufbar
- den Tierbesitzer, d.h. alle von einem Besitzer bzw. einer Herde vermarkteten Tiere können gepoolt werden
- die Eltern des Tieres, d.h. nach einer mehrjährigen Erfassung aller geborenen Rinder kann über die Datei automatisch die Elternschaft und auch die Zahl der Nachkommen pro Eltern überprüft werden.

Eine Individualtypisierung von Nutztieren einer ganzen Population ist ein Novum. Jeder Konsument, Gast, Kunde, Händler, Metzger, Verarbeiter, Besitzer oder Kontrolleur usw. könnte die Identität und damit Herkunft eines Tieres oder Tierproduktes überprüfen lassen (Fig. 4). Notwendig für diese Überprüfung ist lediglich die Entnahme einer ZweitProbe aus beispielsweise dem Schlachthof, dem Supermarkt und sogar aus dem bereits zubereiteten Mahl (beispielsweise einem im Restaurant aufgetischten Wiener Schnitzel oder Tafelspitz).

Bei lebenden Tieren z.B. bei der Überwachung der Tiergerechtheit von Transporten (Entfernung) wären ebenfalls eine Typisierung unzweifelhaft zu ermöglichen. Dieses Maximum an Sicherheit bei der Überprüfung wird automatisch zur Folge haben, daß bei ausreichend großer Überprüfungsfrequenz Betrugsversuche wegen der Furcht vor einem zuverlässigen und gerichtlich einwandfreien Nachweis- bzw. Aufdeckungsmöglichkeit vorab bereits auf ein Minimum reduziert werden.

Bei allen zukünftig möglicherweise noch auftretenden Anordnungen der Überwachung von bestimmten Gruppen von Tieren oder Beständen aufgrund von Risikoabwendungen, Seuchenüberwachung oder Überprüfung von unerlaubten Rückständen (Hormone, Wachstumsbeschleuiniger etc.) in Tierkörpern könnte anhand der dann bereits vorhandenen DNA-Daten ad hoc vorgegangen werden.

Wenn diese Individualtypisierung mit dem hier beschriebenen System in einem Land konsequent praktiziert wird, wird man bei auftretenden Problemen (z.B. BSE-Verdacht bei einem Tier, Ausbruch der Schweinepest in einem Betrieb etc.) jederzeit in der Lage sein, jeweils unmittelbar, d.h. noch am selben Tag, zu reagieren und damit für die Verbraucher einen einzigartigen Schutz oder für die Überwachung eine einzigartige Kontrollmöglichkeit zu bieten.

Insbesondere könnte z.B. durch die Individualtypisierung auch lückenlos und zweifelsfrei bewiesen werden, daß Tiere und Produkte aus alternativer Produktion wirklich dorther stammen. Das bedeutet, daß jeder Kunde auch noch nach weitläufigsten Transporten und Vermarktungen des Fleisches durch Einsendung von Proben (Zweitprobe) eine Überprüfung der das Fleisch begleitenden Informationen veranlassen könnte.

Die Erfindung wird nun anhand von Beispielen näher erläutert, die nicht zur Begrenzung der in den Ansprüchen niedergelegten Erfindung gedacht sind.

### Beispiel 1

### Individualtypisierung beim Rind

Ein Rind wurde mit herkömmlichen Ohrmarken gekennzeichnet. Auf den Dorn der Dornplatte wurde das Probengewinnungsmittel aufgesetzt, das die Form eines zu dem vorderen Ende hin konisch zulaufenden Kegels mit einem zylindrischen Basisteil und einer Vertiefung im Basisteil zur Aufnahme des Dorns der Dornplatte ausgebildet war.

An der Zange wurde zudem der mit einer Lasche einstückig ausgebildete Probenaufnahmebehälter unter der für die Lochplatte vorgesehenen Position derart angeordnet, daß der aus einer Kunststoffolie bestehende Deckel des Probenaufnahmebehälter mit dem Loch der Lochplatte ausgerichtet war.

Die Lasche wies an einer von dem Ort der Probenaufnahmebehälter entfernt liegenden Stelle ein Durchgangsloch zur ortststabilen Befestigung der Lasche an der Zange auf.

Durch Hindurchdrücken des mit dem Probengewinnungsmittel versehenen Dorns der Dornplatte durch das Ohr des Rindes wurde die dabei aus dem Ohr ausgestanzte Gewebeprobe in dem Probenaufnahmebehälter gedrückt. Das Probengewinnungsmittel wurde durch auf den Innenwänden des im Probenaufnahmebehälter vorgesehenen Vorsprüngen nach dem Einführen fixiert, wobei der gebildete Probenraum dichtend verschlossen wurde.

In dem Probenaufnahmebehälter befand sich ein Molekularsieb (Merck 0,2 nm Nr. 1.05704.0250), das die DNA der aufgenommenen Gewebeprobe vor einem Abbau schützt.

Mit dem in dem Probenaufnahmebehälter vorhandenen genetischen Material konnte nach einem halben Jahr Lagerung problemlos DNA-Footprints, sowie PCR-Analysen auf bekannte Gene durchgeführt werden.

### Beispiel 2

Screen-out von funktionellen Mutanten zur Zucht spezieller Linien (z.B. von 1.3GalαGal3 negativen Schweinen für die Xenotransplantation

In einer genügend großen Population beinhaltet jedes im Genom vorhandene Gen, bedingt durch die bekannte natürliche Mutationsrate, bei einzelnen Individuen nicht nur verschiedene Silent-Mutationen, sondern mit einer gewissen Wahrscheinlichkeit auch Mutationen, die eine Inaktivität des Allels zur Folge haben. In der Mehrzahl dieser Fälle handelt es sich dabei nicht um dominante sondern um rezessive Mutationen. Gemäß Hardy-Weinberg-Regel ist die überwiegende Zahl dieser mutationsbedingten Veränderungen im Genotyp von heterozygoten Individuen maskiert.

Entscheidend ist, durch geeignete molekulargenetische Analysen des entsprechenden Genortes in den vorhandenen Schweine-Populationen in einem Screening-Verfahren ein (einziges) heterozygotes Anlageträgertier, das eine inaktivierende Mutation enthält, zu entdecken. Mit diesem Tier kann dann eine homozygot negative Linie aufgebaut werden, die den gleichen gewünschten und benötigten Gendefekt aufweisen würde, wie eine durch Gen-Knock-out generierte Linie. Sie würde dieser in nichts nachstehen.

Die vorgeschlagene Vorgehensweise entspricht gewissermaßen dem Screenen einer Stammzellinie nach Rekombination mit einem geeigneten Konstrukt, das keinen selektiven Marker enthält. Der Unterschied ist folgender: Alle Zellen einer Stammzellinie haben den gleichen Genotyp mit Ausnahme derjenigen, die nach dem einmaligen Mutationsereignis eine Veränderung bzw. Rekombination aufweisen. Beim Screenen von Schweinepopulationen sind alle analysierten Genotypen verschieden. Sie sind nicht gezielt mutagenisiert worden, tragen aber Mutationen, die sich im Laufe der Evolution und züchterischen Bearbeitung angehäuft haben (soweit sie im heterozygoten Zustand keinem negativen selektiven Druck ausgesetzt waren).

Es ist bekannt, daß es quantitative Unterschiede bei der 1.3GalαGal3 Expression gibt. Da die Mutationsanalyse darauf gerichtet ist, in den diversen Populationen schon vorhandene Mutationen zu finden, werden Zuchttiere untersucht. Vernachlässigt man Neumutationen, dann können Mastschweine nur Mutationen tragen, die bei den Zuchteltern schon vorhanden sind. Für die praktische Durchführung ist die Asservierung und Sammlung der Gewebeproben mit dem Typi-Fix-System ein entscheidender Faktor für den Erfolg bzw. eine finanziell machbare Realsierung. Bei der Analyse zur Suche nach Mutationsträgern muß davon ausgegangen werden, daß möglicherweise bis zu 100.000 Genotypen untersucht werden müssen, um potentielle Tiere mit einer 1.3GalaGal Defizienz zu finden. Für das im Prinzip sehr aufwendige Sammeln der Individualroben von 100.000 Schweinen ist das erfindungsgemäße System das Verfahren der Wahl. Die Proben brauchen nur noch (per Post oder durch Sammelaktionen) ins Labor transportiert und dort weiterbearbeitet zu werden.

## Patentansprüche

1. Vorrichtung zur Gewinnung und Erst-Aufbereitung einer DNA-haltige Zellen enthaltenden Probe aus einem Tier, umfassend,
einen Probenaufnahmebehälter (1), und
Mittel zum Gewinnen der Probe (4), das nach Gewinnen der Probe in den Probenaufnahmebehälter eingeführt wird und diesen dichtend verschließt,
wobei
der Probenaufnahmebehälter (1) ein Bodenteil (2) und Seitenwände (3) aufweist und in einem von dem Boden entfernt liegenden Bereich der Behälterseitenwände Mittel (5) zum Fixieren des eingeführten Probengewinnungsmittel besitzt, wobei der Probenaufnahmebehälter mit einem leicht durchdringbaren Deckel versehen ist und in dem Behälter Mittel zum Schutz vor DNA abbauenden Enzymen vorgesehen sind,
das Probengewinnungsmittel (4) derart ausgestaltet ist, daß damit in einem Arbeitsgang die Probe gewonnen und in den Probenaufnahmebehälter eingeführt wird, und das Probengewinnungsmittel (4) mit den am Probenaufnahmebehälter vorgesehenen Mittel zum Fixieren (5) ortstabil befestigt wird und den Probenbehälter in mindestens einen Probenraum (6, 6a) einteilt, der durch den Boden (2) und die Seitenwände (3) des Probenaufnahmebehälters (1) und das vordere Ende des Probengewinnungsmittels (7) begrenzt wird, und wobei
der Probenaufnahmebehälter (1) mit einer zur Identifikationsmarkierung des Tieres einsetzbaren, eine Lochplatte (11) und eine Dornplatte (10) aufweisenden, und mit einer Markierung versehenen Ohrmarke assoziiert ist, wobei der Probenaufnahmebehälter (1) lösbar mit der Lochplatte (11) der Ohrmarke verbunden ist und mit einer gleichen Markierung versehen ist wie die Ohrmarke, und wobei das Probengewinnungsmittel (4) lösbar mit der Dornplatte (10) der Orhmarke verbunden ist.

2. Vorrichtung nach Anspruch 1, **dadurch gekennzeichnet, daß** das Probengewinnungsmittel (4) auf den Dorn (10a) der Dornplatte aufgesetzt ist.

3. Vorrichtung nach einem der Ansprüche 1 oder 2, **dadurch gekennzeichnet, daß** die Markierung eine Identifikationsnummer ist.

4. Vorrichtung nach einem der vorhergehenden Ansprüche, **dadurch gekennzeichnet, daß** das Probengewinnungsmittel (4) an dem dem Probenaufnahmebehälterboden zugewandten Ende mindestens eine scharfe Kante aufweist.

5. Vorrichtung nach einem der vorhergehenden Ansprüche, **dadurch gekennzeichnet, daß** sich das Probengewinnungsmittel (4) in Richtung auf das dem Probenaufnahmebehälterboden zugewandte Ende hin verjüngt.

6. Vorrichtung nach einem der vorhergehenden Ansprüche, wobei am Boden (2) des Probenaufnahmebehälters (1) ein in den Behälterinnenraum ragender Vorsprung (8) bereitgestellt wird und das Probengewinnungsmittel (4) an dessen dem Probenaufnahmebehälterboden zugewandten Ende (7) zur Aufnahme des Vorsprungs angepaßt ist, so daß das von dem Probengewinnungsmittel (4) eingebrachte Probenmaterial zwischen dem Vorsprung (8) und dem Probengewinnungsmittel (4) zerkleinert wird.

7. Vorrichtung nach Anspruch 6, wobei der Vorsprung (8) mit den Seitenwänden (3) des Probenaufnahmebehälters (1) derart verbunden ist, daß nach Einführen eines entsprechend angepaßten Probengewinnungsmittels (4) der Probenaufnahmebehälter (1) in zwei Probenräume (6, 6a) unterteilt wird.

8. Vorrichtung nach Anspruch 6 oder 7, wobei der Vorsprung (8) eine Kegelform umfaßt.

9. Vorrichtung nach einem der Ansprüche 6 bis 8, wobei der Vorsprung (8) eine den Probenaufnahmebehälter in zwei Bereiche unterteilende Wand ist

10. Vorrichtung nach einem der vorhergehenden Ansprüche, wobei der Probenaufnahmebehälter (1) an einer Haltevorrichtung (9) befestigt ist.

11. Vorrichtung nach Anspruch 10, wobei die Haltevorrichtung (9) eine Lasche ist.

12. Vorrichtung nach einem der vorhergehenden Ansprüche, wobei das Probengewinnungsmittel an dessen dem Probenaufnahmebehälterboden abgewandten Ende zur Aufnahme eines Stiftes ausgebildet ist.

13. Vorrichtung nach einem der vorhergehenden Ansprüche, wobei das Mittel zum Schutz vor DNA-abbauenden Enzymen, Lauge, Proteinase K oder ein Molekularsieb ist.

14. Vorrichtung nach Anspruch 13, wobei die Proteinase K von einem geeigneten Puffer durch eine Membran getrennt ist, die beim Einführen des Probengewinnungsmittels zerstört wird, so daß die Proteinase K mit dem Puffer in Kontakt gebracht wird.

15. Verfahren zur Gewinnung einer DNA-haltige Zellen enthaltenden Probe aus einem Tier mittels einer, einen Probenaufnahmebehälter (1) und ein Probengewinnungsmittel (4) aufweisenden Vorrichtung gemäß einer der Ansprüche 1-14, wobei in einem Arbeitsgang und unter gleichzeitiger Durchführung einer Ohrmarkierung des Tieres mit einer Ohrmarke mit dem dem Probenaufnahmebehälterboden zugewandten Ende (7) des Probengewinnungsmittels (4) eine geeignete Probe dadurch gewonnen wird, daß das Probengewinnungsmittel (4) mit einer geeigneten Vorrichtung durch das Ohr eines Tiers gedrückt und in den Probenaufnahmebehälter (1) eingeführt und darin ortstabil fixiert wird, so daß ein durch den Boden (2) und die Seitenwände (3) des Probenaufnahmebehälters (1) und den dem Probenaufnahmebehälterboden zugewandten Teil (7) des Probengewinnungsmittels (4) begrenzter Probenraum (6, 6a) gebildet wird, der gegenüber der Umgebung dicht abgeschlossen ist, und wobei zur Identifikation des Tieres der Probenaufnahmebehälter (1) mirt der gleichen Markierung versehen wird, wie die Ohrmarke.

16. Verwendung der Vorrichtung nach einem der Ansprüche 1-14 in einem Verfahren zur Typisierung von Tierpopulationen, wobei Proben mit der Vorrichtung gewonnen, in einem Labor analytisch untersucht und katalogisiert werden.

17. Verwendung einer Vorrichtung nach einem der Ansprüche 1 bis 14 zur Gewinnung einer DNA-haltige Zellen enthaltenden Probe aus einem Tier.

## Claims

1. A device for collecting and initially preparing samples comprising DNA containing cells from an animal, comprising
a sample receiving container (1), and
sample collecting means (4), which means enters the sample receiving container after collecting the sample and tightly seals it, wherein
the sample receiving container (1) has a base (2) and side walls (3), has on the container's side walls, in an area removed from the base, means (5) for securing the introduced sample collecting means, and is provided with an easily penetrable lid, and provided in the container are means to protect from DNA-degrading enzymes, and wherein
the sample collecting means (4) is made in such a way that in one step the sample is collected and introduced into the sample receiving container, and that it on entry into the sample receiving container is fixed in place by the means for securing (5) and divides the sample receiving container into at least one sample space (6, 6a), which is limited by the base (2) and the side walls (3) of the sample receiving container (1) and the front end of the sample collecting means (7), and wherein
the sample receiving container (1) is associated with an ear tag, which is provided with a means of verifying identity and comprising an aperture plate (11) and a spike plate (10), and which may be used for the identification of an animal, wherein the sample collection container (1) is releasable connected with the aperture plate (11) of the ear tag and is provided with the same means of verifying identity as the ear tag, and wherein the sample collecting means (4) is releasable connected with the spike plate (10) of the ear tag.

2. The device according to claim 1, **characterized in that** the sample collecting means (4) is placed on the spike (10a) of the spike plate.

3. The device according to any of the claims 1 or 2, **characterized in that** the tag is an identification number.

4. The device according to any of the preceding claims, **characterized in that** the sample collecting means (4) has at least one sharp edge at the end facing the base of the sample receiving container.

5. The device according to any of the preceding claims, **characterized in that** the sample collecting means (4) tapers at the end facing the base of the sample receiving container.

6. The device according to any of the preceding claims, wherein the base (2) of the sample receiving container (1) has a projection (8) into the container's internal space and the sample collecting means (4) on its end (7) facing the base of the sample receiving container is suited to accept the projection, so that the sample material introduced by the sample collecting means (4) is crushed between the projection (8) and the sample collection means (4).

7. The device according to claim 6, wherein the projection (8) is connected in such a way with the side walls (3) of the sample receiving container (1), that after introduction of a suitable sample collecting means (4) the sample receiving container (1) is divided into 2 sample spaces (6,6a).

8. The device according to claim 6 or 7, wherein the projection (8) comprises a cone form.

9. The device according to any of the claims 6 to 8, wherein the projection (8) is a wall dividing the sample receiving container into 2 areas.

10. The device according to any of the preceding claims, wherein the sample receiving container (1) is attached to a holding device (9).

11. The device according to claim 10, wherein the holding device (9) is a tongue.

12. The device according to any of the preceding claims, wherein the sample collecting means is formed at the end opposite to the base of the sample receiving container such as to allow for the insertion of a rod at its rear side.

13. The device according to any of the preceding claims, wherein the means protecting from DNA degrading enzymes is alkali, proteinase K or a molecular sieve.

14. The device according to claim 13, wherein the proteinase K is separated from a suitable buffer by a membrane, which is destroyed on entry of the sample collecting means, so that the proteinase K is brought into contact with the buffer.

15. A process for collecting a sample comprising DNA-containing cells from an animal by means of a device according to any of the claims 1 to 14, comprising a sample receiving container (1) and a sample collecting means (4), wherein in one step and at the same time as performing the ear tagging of the animal with an ear tag, with the end of the sample collecting means (7) facing the base of the sample receiving container a suitable sample is collected by pressing the sample collecting means (4) with an appropriate device through the ear of an animal and introducing it into the sample receiving container (1) and securing it there, such that a sample space (6, 6a) is formed, limited by the base (2) and the side walls (3) of the sample receiving container (1) and the end (7) of the sample collecting means (4) facing the base of the sample receiving container, which is tightly sealed from the environment, and wherein, for identifying the animal, the sample receiving container (1) is provided with the same tag as the ear tag.

16. Use of a device according to any of the claims 1 to 14 in a process for typing animal populations, wherein samples are collected with the device and are analytically examined and classified in a laboratory.

17. Use of a device according to any of the claims 1 to 14 for the collection of a sample comprising DNA containing cells from an animal.

## Revendications

1. Dispositif pour l'obtention et pour la première préparation d'un échantillon d'un animal, ledit échantillon contenant des cellules contenant de l'ADN, ledit dispositif comprenant :
un récipient de réception d'échantillon (1), et
un moyen pour l'obtention d'échantillon (4), qui est introduit dans le récipient de réception d'échantillon, après l'obtention de l'échantillon, et ferme celui-ci de façon étanche,
le récipient de réception d'échantillon (1) ayant un élément de fond (2) et des parois latérales (3) et ayant des moyens (5) pour fixer le moyen d'obtention d'échantillon étant introduit, situés dans une zone des parois latérales, éloignée du fond, le récipient de réception d'échantillon étant pourvu d'un couvercle facilement pénétrable, et des moyens pour protéger contre des enzymes ADN-dégradants étant pourvus dans le récipient,
le moyen d'obtention d'échantillon (4) étant formé d'une telle manière, que l'échantillon est obtenu et est introduit grâce à celui-ci pendant une étape dans le récipient de réception d'échantillon et que le moyen d'obtention d'échantillon (4) est fixé de manière fixe avec les moyens fixants (5) pourvus sur le récipient de réception d'échantillon et divise le récipient d'échantillon en au moins un compartiment d'échantillon (6, 6a), qui est limité par le fond (2) et les parois latérales (3) du récipient de réception d'échantillon (1) et l'extrémité avant (7) du moyen d'obtention d'échantillon, et
le récipient de réception d'échantillon (1) étant associé avec une marque d'oreille utilisable pour le marquage d'identification de l'animal, la marque d'oreille ayant une plaque perforée (11) et une plaque à mandrin (10) et étant pourvue d'un marquage, le récipient de réception d'échantillon (1) étant lié de manière détachable avec la plaque perforée (11) de la marque d'oreille et étant pourvu du même marquage que la marque d'oreille, le moyen d'obtention d'échantillon (4) étant lié de manière détachable avec la plaque à mandrin (10) de la marque d'oreille.

2. Dispositif selon la revendication 1, **caractérisé en ce que** le moyen d'obtention d'échantillon (4) est placé sur le mandrin (10a) de la plaque à mandrin.

3. Dispositif selon l'une des revendications 1 ou 2, **caractérisé en ce que** le marquage est un numéro d'identification.

4. Dispositif selon l'une des revendications précédentes, **caractérisé en ce que** le moyen d'obtention d'échantillon (4) a à l'extrémité étant en face du fond du récipient de réception d'échantillon au moins un bord acéré.

5. Dispositif selon l'une des revendications précédentes, **caractérisé en ce que** le moyen d'obtention d'échantillon (4) se rétrécit en direction vers l'extrémité étant en face du fond du récipient de réception d'échantillon.

6. Dispositif selon l'une des revendications précédentes, une saillie (8) étant mise à disposition au fond (2) du récipient de réception d'échantillon (1), ladite saillie saillant vers l'intérieur du récipient, et le moyen d'obtention d'échantillon (4) étant adapté, à son extrémité (7) étant en face du fond du récipient de réception d'échantillon, à recevoir la saillie, de manière que le matériau d'échantillon étant introduit par le moyen d'obtention d'échantillon (4) soit broyé entre la saillie (8) et le moyen d'obtention d'échantillon (4).

7. Dispositif selon la revendication 6, la saillie (8) étant reliée d'une telle manière avec les parois latérales (3) du récipient de réception d'échantillon (1), que le récipient de réception d'échantillon (1) est divisé en deux compartiments d'échantillon (6, 6a) après l'insertion d'un moyen d'obtention d'échantillon (4) adapté de façon appropriée.

8. Dispositif selon la revendication 6 ou 7, la saillie (8) comprenant une forme conique.

9. Dispositif selon l'une des revendications 6 à 8, la saillie (8) étant une paroi divisant le récipient de réception d'échantillon en deux zones.

10. Dispositif selon l'une des revendications précédentes, le récipient de réception d'échantillon (1) étant fixé à un dispositif de retenue (9).

11. Dispositif selon la revendication 10, le dispositif de retenue (9) étant une attache.

12. Dispositif selon l'une des revendications précédentes, le moyen d'obtention d'échantillon, à son extrémité étant détournée du fond du récipient de réception d'échantillon, est formé d'une telle manière à recevoir une broche.

13. Dispositif selon l'une des revendications précédentes, le moyen pour protéger contre des enzymes ADN-dégradants étant une lessive, protéinase K ou un tamis moléculaire.

14. Dispositif selon la revendication 13, la protéinase K étant séparée d'un tampon approprié par une membrane, qui sera détruite en introduisant le moyen d'obtention d'échantillon, de manière que la protéinase K soit mise en contact avec le tampon.

15. Procédé d'obtention d'un échantillon d'un animal, ledit échantillon contenant des cellules contenant de l'ADN, au moyen d'un dispositif selon l'une des revendications 1-14 avec un récipient de réception d'échantillon (1) et un moyen d'obtention d'échantillon (4), dans quel procédé, pendant une étape et pendant que simultanément un marquage d'oreille de l'animal est réalisé avec une marque d'oreille, un échantillon approprié est obtenu par l'extrémité (7) du moyen d'obtention d'échantillon (4) située en face du fond du récipient de réception d'échantillon, en perçant le moyen d'obtention d'échantillon (4) avec un dispositif approprié à travers l'oreille de l'animal et en introduisant ledit moyen d'obtention d'échantillon dans le récipient de réception d'échantillon (1) et en fixant ledit moyen d'obtention d'échantillon de manière fixe dedans, de telle manière qu'un compartiment d'échantillon (6, 6a) limité par le fond (2) et par les parois latérales (3) du récipient de réception d'échantillon (1) et par la partie (7) du moyen d'obtention d'échantillon (4) en face du fond du récipient de réception d'échantillon est formé, ledit compartiment d'échantillon étant fermé de façon étanche par respect à l'environnement, et le récipient de réception d'échantillon (1) étant pourvu du même marquage que la marque d'oreille pour l'identification de l'animal.

16. Utilisation du dispositif selon l'une des revendications 1-14, dans un procédé pour typer des populations animales, dans lequel des échantillons sont obtenus par le dispositif, sont analysés dans un laboratoire et sont catalogués.

17. Utilisation d'un dispositif selon l'une des revendications 1-14 pour l'obtention d'un échantillon d'un animal, ledit échantillon contenant des cellules contenant de l'ADN.
